(19) Europäisches Patentamt
European Patent Office
Office européen des brevets

(11) Veröffentlichungsnummer: **0 308 773**
**A2**

(12) # EUROPÄISCHE PATENTANMELDUNG

(21) Anmeldenummer: **88114915.7**

(51) Int. Cl.4: **C07D 249/08 , A01N 43/653**

(22) Anmeldetag: **13.09.88**

(30) Priorität: **23.09.87 DE 3731927**

(43) Veröffentlichungstag der Anmeldung: **29.03.89 Patentblatt 89/13**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Krämer, Wolfgang, Dr.**
**Rosenkranz 25**
**D-5093 Burscheid 2(DE)**
Erfinder: **Holmwood, Graham, Dr.**
**Krutscheider Weg 105**
**D-5600 Wuppertal 11(DE)**
Erfinder: **Büchel, Karl Heinz, Prof. Dr.**
**Dabringhausener Strasse 42**
**D-5093 Burscheid(DE)**
Erfinder: **Dutzmann, Stefan, Dr.**
**Leinenweberweg 33**
**D-4000 Düsseldorf 13(DE)**
Erfinder: **Brandes, Wilhelm, Dr.**
**Eichendorffstrasse 3**
**D-5653 Leichlingen(DE)**
Erfinder: **Lürssen, Klaus, Dr.**
**August-Kierspel-Strasse 145**
**D-5060 Bergisch Gladbach 2(DE)**

(54) **Substituierte Triazolylalkanole.**

(57) Neue substituierte Triazolylalkanole der Formel

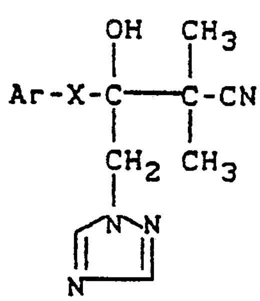

(I)

in welcher
Ar für gegebenenfalls substituiertes Aryl steht und
X für eine der Gruppen $-CH_2$, $-O-CH_2-$, $-S-CH_2$, $-CH_2-CH_2-$, $-CH=CH-$ oder $-C\equiv C-$ steht,
sowie deren Säureadditionssalze und Metallsalzkomplexe, ein Verfahren zur Herstellung der neuen Stoffe und deren Verwendung als Fungizide und Pflanzenwachstumsregulatoren.

EP 0 308 773 A2

## Substituierte Triazolylalkanole

Die Erfindung betrifft neue substituierte Triazolylalkanole, ein Verfahren zu ihrer Herstellung sowie ihre Verwendung als Fungizide und Pflanzenwachstumsregulatoren.

Es ist bekannt, daß bestimmte substituierte Triazolylalkanole fungizide und pflanzenwuchsregulierende Eigenschaften besitzen (vgl. DE-OS 3 018 866 und DE-OS 3 202 601). So lassen sich zum Beispiel das 2,2-Dimethyl-5-(4-fluorphenyl)-3-(1,2,4-triazol-1-ylmethyl)-pentan-3-ol, das 3-(4-Chlorphenoxy)-3-methyl-1-(4-phenylphenoxy)-2-(1,2,4-triazol-1-ylmethyl)-butan-2-ol, das 5-(4-Chlorphenyl)-2,2-dimethyl-1-methoxy-3-(1,2,4-triazol-1-ylmethyl)-pentan-3-ol oder das 5-(2,4-Dichlorphenyl)-2,2-dimethyl-1-fluor-3-(1,2,4-triazol-1-ylmethyl)-pentan-3-ol zur Bekämpfung von Pilzen sowie zur Regulierung des Pflanzenwachstums verwenden. Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden nun neue substituierte Triazolylalkanole der Formel

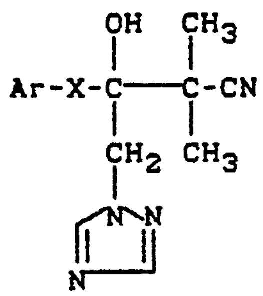

(I)

in welcher

Ar für gegebenenfalls substituiertes Aryl steht und

X für eine der Gruppen $-CH_2-$, $-O-CH_2-$, $-S-CH_2-$, $-CH_2-CH_2-$, $-CH=CH-$ oder $-C\equiv C-$ steht,

sowie deren Säureadditionssalze und Metallsalzkomplexe gefunden.

Die Verbindungen der Formel (I) enthalten ein asymmetrisch substituiertes Kohlenstoffatom und können deshalb in den beiden optischen Isomerenformen oder auch als Isomerengemische unterschiedlicher Zusammensetzung anfallen. Die Erfindung betrifft sowohl die Racemate als auch die einzelnen Isomeren und deren Gemische.

Außerdem können die erfindungsgemäßen Verbindungen der Formel (I), in denen C für $-CH=CH-$ steht, in den geometrischen Isomerenformen cis und trans vorkommen. Die Erfindung betrifft sowohl die einzelnen Isomeren als auch deren Gemische.

Weiterhin wurde gefunden, daß man substituierte Triazolylalkanole der Formel (I) sowie deren Säureadditionssalze und Metallsalzkomplexe erhält, wenn man Oxim-Derivate der Formel (II),

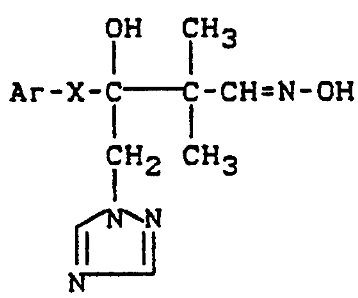

(II)

in welcher

Ar und X die oben angegebene Bedeutung haben,

mit einem wasserabspaltenden Mittel, gegebenenfalls in Gegenwart eines Verdünnungsmittels und gegebenenfalls eines Reaktionshilfsmittels, umsetzt und gegebenenfalls anschließend an die so erhaltenen substituierten Triazolylalkanole der Formel (I) eine Säure oder ein Metallsalz addiert.

Schließlich wurde gefunden, daß die neuen substituierten Triazolylalkanole der Formel (I) sowie deren Säureadditions-Salze und Metallsalz-Komplexe sehr gute fungizide und pflanzenwuchsregulierende Eigenschaftten besitzen.

Überraschenderweise zeigen die erfindungsgemäßen Stoffe eine erheblich bessere fungizide Wirksamkeit als das 2,2-Dimethyl-5-(4-fluorphenyl)-3-(1,2,4-triazol-1-ylmethyl)-pentan-3-ol, das 3-(4-Chlorphenoxy)-3-methyl-1-(4-phenylphenoxy)-2-(1,2,4-triazol-1-ylmethyl)-butan-2-ol, das 5-(4-Chlorphenyl)-2,2-dimethyl-1-methoxy-3-(1,2,4-triazol-1-ylmethyl)-pentan-3-ol oder das 5-(2,4-Dichlorphenyl)-2,2-dimethyl-1-fluor-3-(1,2,4-triazol-1-ylmethyl)-pentan-3-ol, welches konstitutionell ähnliche, vorbekannte Wirkstoffe gleicher Wirkungsrichtung sind.

Die erfindungsgemäßen substituierten Triazolylalkanole sind durch die Formel (I) allgemein definiert. Bevorzugt sind Verbindungen der Formel (I), in denen

Ar für Phenyl steht, das einfach oder mehrfach, gleichartig oder verschieden substituiert sein kann durch Halogen, Nitro, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, wie Fluor-, Chlor- und Bromatomen, geradkettiges oder verzweigtes Halogenalkoxy mit 1 bis 4 Kohlenstoffatomen und 1 bis 9 gleichen oder verschiedenen Halogenatomen, wie Fluor-, Chlor- und Bromatomen, geradkettiges oder verzweigtes Halogenalkylthio mit 1 bis 4 Kohlenstoffatomen und 1 bis 9 gleichen oder verschiedenen Halogenatomen, wie Fluor-, Chlor- und Bromatomen, durch gegebenenfalls ein- oder mehrfach, gleichartig oder verschieden durch Halogen substituiertes Phenyl und/oder gegebenenfalls ein- oder mehrfach, gleichartig oder verschieden durch Halogen substituiertes Phenoxy, oder

Ar für gegebenenfalls einfach oder mehrfach, gleichartig oder verschieden durch Halogen und/oder Alkyl mit 1 bis 4 Kohlenstoffatomen substituiertes Naphthyl steht und

X für die Gruppierungen $-CH_2-$, $-OCH_2-$, $-SCH_2-$, $-CH_2-CH_2-$, $-CH=CH-$ oder $-C\equiv C-$ steht, wobei das Heteroatom mit dem Arylrest verbunden ist, wenn X für $-OCH_2-$ oder $-SCH_2-$ steht.

Ganz besonders bevorzugt sind Verbindungen der Formel (I), in denen

Ar für Phenyl steht, das einfach bis dreifach, gleichartig oder verschieden substituiert sein kann durch Fluor, Chlor, Brom, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, gegebenenfalls einfach bis dreifach, gleichartig oder verschieden durch Fluor, Chlor und/oder Brom substituiertes Phenyl und/oder gegebenenfalls einfach bis dreifach, gleichartig oder verschieden durch Fluor, Chlor und/oder Brom substituiertes Phenoxy, oder

Ar für gegebenenfalls einfach bis dreifach, gleichartig oder verschieden durch Fluor, Chlor, Brom und/oder Methyl substituiertes Naphthyl steht und

X für die Gruppierungen $-CH_2-$, $-OCH_2-$, $-SCH_2-$, $-CH_2-CH_2-$, $-CH=CH-$ oder $-C\equiv C-$ steht, wobei das Heteroatom mit dem Arylrest verbunden ist, wenn X für $-OCH_2-$ oder $-SCH_2-$ steht.

Bevorzugte erfindungsgemäße Verbindungen sind auch Additionsprodukte aus Säuren und denjenigen substituierten Triazolylalkanolen der Formel (I), in denen die Substituenten Ar und X die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen substituierten Triazolylalkanolen der Formel (I), in denen die Substituenten Ar und X die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Im einzelnen seien außer den bei den Herstellungsbeispielen genannten Verbindungen die folgenden substituierten Triazolylalkanole der Formel (I) genannt:

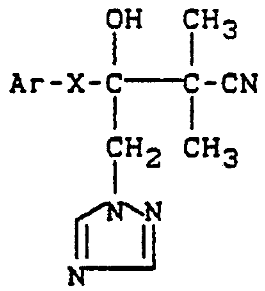

(I)

| Ar | X | Ar | X |
|---|---|---|---|
| 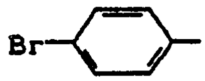 | $-O-CH_2-$ | 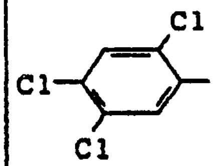 | $-CH_2-CH_2-$ |
| 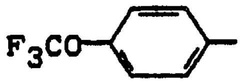 | $-CH=CH-$ | 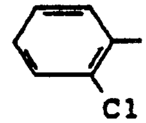 | $-CH_2-CH_2-$ |
| 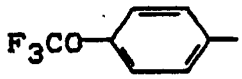 | $-CH_2-CH_2-$ | 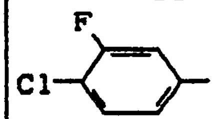 | $-CH_2-CH_2-$ |
| 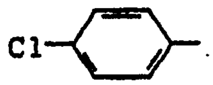 | $-C\equiv C-$ | 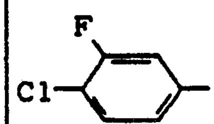 | $-CH=CH-$ |
| 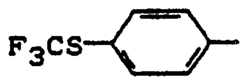 | $-O-CH_2-$ | 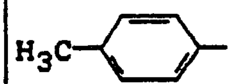 | $-CH_2-$ |
| 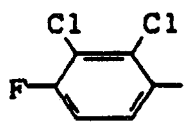 | $-O-CH_2-$ | 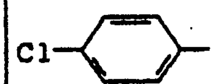 | $-CH_2-$ |
| 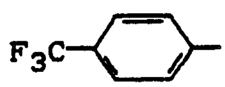 | $-CH_2-CH_2-$ | | |
| 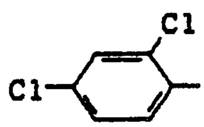 | $-CH_2-$ | 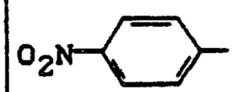 | $-CH_2-$ |
| 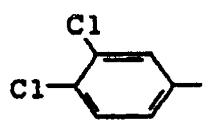 | $-CH_2-$ | 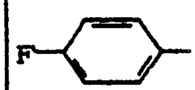 | $-CH_2-$ |
| 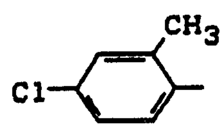 | $-O-CH_2-$ | | |

Verwendet man beispielsweise 2,2-Dimethyl-3-(4-chlor-2-fluorbenzyl)-3-hydroxy-4-(1,2,4-triazol-1-yl)-butanal-oxim als Ausgangsverbindung und Acetanhydrid als wasserabspaltendes Mittel, so läßt sich der Reaktionsablauf des erfindungsgemäßen Verfahrens durch das folgende Formelschema darstellen:

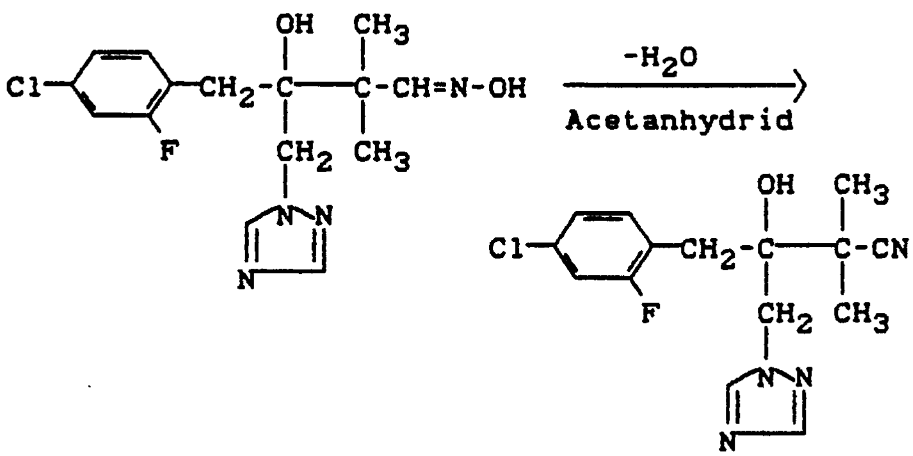


Die zur Durchführung des erfindungsgemäßen Verfahrens als Ausgangsstoffe benötigten Oxim-Derivate sind durch die Formel (II) allgemein definiert. In dieser Formel (II) stehen Ar und X vorzugsweise für diejenigen Reste, die bereits im Zusammenhang mit der Beschreibung der erfindungsgemäßen Stoffe der Formel (I) als bevorzugt für diese Substituenten genannt wurden.

Die Oxim-Derivate der Formel (II) sind bekannt oder können nach bekannten Verfahren erhalten werden (vergl. DE-OS 33 34 779 sowie die Herstellungsbeispiele).

Das erfindungsgemäße Verfahren wird in Gegenwart eines geeigneten wasserabspaltenden Mittels durchgeführt. Als solche kommen alle üblichen Dehydratisierungsmittel in frage (vergl. hierzu beispielsweise C.Ferri "Reaktionen der organischen Synthese", S.572; Thieme Verlag, Stuttgart 1978). Mit besonderem Vorzug verwendet man Carbonsäureanhydride, wie beispielsweise Acetanhydrid.

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Ester, wie Essigsäureethylester.

Es ist auch möglich, bei Verwendung flüssiger, wasserabspaltender Mittel, diese in einem entsprechenden Überschuß gleichzeitig als Verdünnungsmittel einzusetzen.

Das erfindungsgemäße Verfahren wird gegebenenfalls in Gegenwart eines geeigneten Reaktionshilfsmittels durchgeführt. Als solche kommen anorganische oder organische Basen in Frage. Hierzu gehören beispielsweise Alkalimetallcarbonate oder -acetate, wie Natriumcarbonat, Natriumacetat, Kaliumcarbonat oder Natriumhydrogencarbonat, sowie tertiäre Amine, wie Triethylamin, N,N-Dimethylanilin, Pyridin oder N,N,Dimethylaminopyridin.

Es ist jedoch auch möglich, das erfindungsgemäße Verfahren ohne Zusatz eines Reaktionshilfsmittels durchzuführen.

Die Reaktionstemperaturen können bei der Durchführung des erfindungsgemäßen Verfahrens in einem größeren Bereich variiert werden. Im allgemeinen arbeitet man bei Temperaturen zwischen 0° C und 120° C, vorzugsweise bei Temperaturen zwischen 40° C und 110° C.

Zur Durchführung des erfindungsgemäßen Verfahrens setzt man pro Mol an Oxim-Derivat der Formel (II) im allgemeinen 1,0 bis 50,0 Mol, vorzugsweise 1,0 bis 10,0 Mol an wasserabspaltendem Mittel und gegebenenfalls 1,0 bis 5,0 Mol, vorzugweise 1,0 bis 2,5 Mol an Reaktionshilfsmittel ein. Die Reaktionsdurchführung, Aufarbeitung und Isolierung der Reaktionsprodukte erfolgt nach allgemein üblichen Methoden.

Die nach dem erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen infrage, die bereits weiter oben beschrieben wurden.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol, und Hinzufügen zu Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können als Fungizide im Planzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Blattfleckenkrankheit der Gerste (Pyrenophora teres) oder gegen den Erreger der Blattfleckenkrankheit des Weizens (Cochliobolus sativus) oder gegen den Erreger der Braunfleckigkeit des Weizens (Leptosphaeria nodorum), gegen Rost- und Mehltauarten sowie zur Bekämpfung von Krankheiten im Obst- und Gemüseanbau, wie beispielsweise gegen den Erreger des echten Gurkenmehltaus (Sphaerotheca fuliginea) oder gegen den Erreger des Apfelschorfs (Venturia inaequalis), gegen Cercosporella- und Uromyces-Arten sowie zur Bekämpfung von Reiskrankheiten, wie beispielsweise gegen den Erreger der Reisfleckenkrankheit (Pyricularia oryzae) einsetzen.

Zu erwähnen ist darüberhinaus eine gute in-vitro-Wirksamkeit der Verbindungen.

Im Materialschutz lassen sich die erfindungsgemäßen Wirkstoffe zum Schutz von technischen Materialien einsetzen. Unter technischen Materialien sind in diesem Zusammenhang nicht lebende Materialien zu verstehen, die für die Verwendung in der Technik zubereitet worden sind. Beispielsweise können technische Materialien, die durch die erfindungsgemäßen Wirkstoffe vor mikrobieller Veränderung oder Zerstörung geschützt werden sollen, Klebstoffe, Leime, Papier, Karton, Textilien, Leder, Holz, Anstrichmittel, Kunststoffartikel, Kühlschmierstoffe und andere Materialien sein, die von Mikroorganismen befallen oder

zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreiskäufe genannt, besonders bevorzugt Holz.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zukkerrohr, Ananas sowie in Zitrusfrüchten zu erhöhen oder den Proteingehalt in Soja oder Getreide zu steigern. Auch ist est beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Frucht fall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Al kylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungiziden, Insektiziden, Akariziden und Herbiziden sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele:

Beispiel 1:

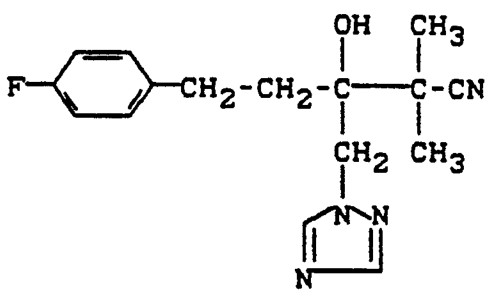

16 g (0,05 Mol) ,2,2-Dimethyl-5-(4-fluorphenyl)-3-hydroxy-3-(1,2,4-triazol-1-ylmethyl)-pentanal-oxim werden in 150 ml Acetanhydrid 3 Stunden auf Rückflußtemperatur erhitzt. Danach wird das Reaktionsgemisch abgekühlt, auf 200 g Eis gegossen und mit 300 ml Dichlormethan extrahiert. Die organische Phase wird über Natriumsulfat getrocknet und unter vermindertem Druck eingeengt. Der Rückstand wird durch Verrühren mit 150 ml Diethylether zur Kristallisation gebracht, abgesaugt und getrocknet.

Man erhält 11g (73 % der Theorie) an 5-(4-Fluorphenyl)-3-hydroxy-2-methyl-3-(1,2,4-triazol-1-ylmethyl)-pentan-2-carbonitril vom Schmelzpunkt 152-154 °C.

Herstellung der Ausgangsverbindungen:

Beispiel II-1

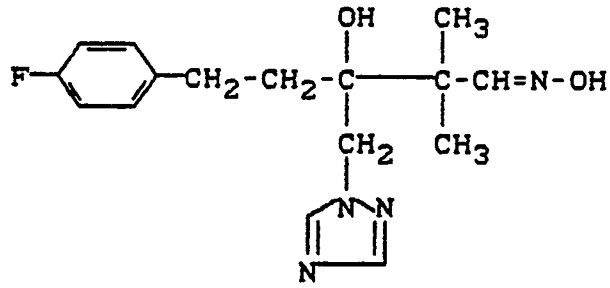

41,2 g (0,135 Mol) ,2,2-Dimethyl-5-(4-fluorphenyl)-3-hydroxy-3-(1,2,4-triazol-1-ylmethyl)-pentanal und 11,1 g (0,16 Mol) Hydroxylaminhydrochlorid werden in 300 ml Ethanol 6 Stunden unter Rückfluß erhitzt. Danach wird das Reaktionsgemisch abgekühlt, in 1000 ml gesättigte wässrige Natriumhydrogencarbonatlösung gegeben und zweimal mit jeweils 300 ml Dichlormethan extrahiert. Die vereinigten organischen Phasen werden zweimal mit jeweils 400 ml Wasser gewaschen, über Natriumsulfat getrocknet und unter vermindertem Druck eingeengt. Der Rückstand wird aus Diisopropylether umkristallisiert.

Man erhält 30 g (70 % der Theorie) an 2,2-Dimethyl-5-(4-fluorphenyl)-3-hydroxy-3-(1,2,4-triazol-1-ylmethyl)-pentanal-oxim vom Schmelzpunkt 101 °C bis 103 °C.

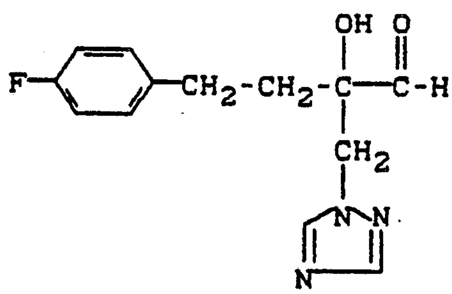

Zu 124 g (0,355 Mol) 2-(1,3-Dioxolan-2-yl)-5-(4-fluorphenyl)-2-methyl-3-(1,2,4-triazol-1-ylmethyl)-pentan-3-ol in einer Mischung aus 850 ml Ethanol und 850 ml Wasser gibt man 85 ml konzentrierte Salzsäure. Man rührt das Reaktionsgemisch 16 Stunden bei Raumtemperatur, engt dann im Wasserstrahlvakuum auf die Hälfte des Volumens ein, neutralisiert mit wässriger Natriumcarbonatlösung, extrahiert mit 600 ml Dichlormethan, wäscht die vereinigten organischen Phasen mit 1500 ml Wasser, trocknet über Natriumsulfat und engt unter vermindertem Druck ein. Der Rückstand wird in 600 ml Aceton aufgenommen. Die Lösung wird bei 0 °C mit 48 g 1,8-Naphthalindisulfonsäure in 80 ml Aceton versetzt und 4 Stunden bei dieser Temperatur gerührt. Der entstandene Niederschlag wird abgesaugt und in 2500 ml gesättigte wässrige Natriumbicarbonatlösung eingerührt. Man extrahiert mehrfach mit insgesamt 600 ml Dichlormethan, trocknet über Natriumsulfat, engt unter vermindertem Druck ein und kristallisiert den Rückstand aus Diisopropylether um.

Man erhält 64 g (59 % der Theorie) an 2,2-Dimethyl-5-(4-fluorphenyl)-3-hydroxy-3-(1,2,4-triazol-1-ylmethyl)-pentanal vom Schmelzpunkt 98 °C.

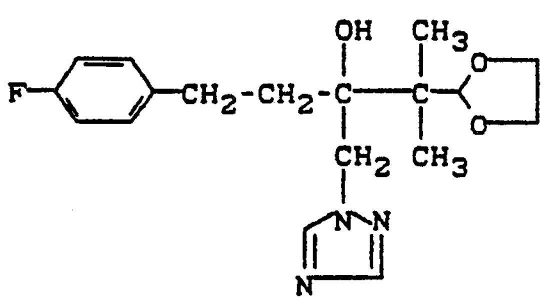

Zu 193 g (0,41 Mol) rohem 2-[2-(1,3-Dioxolan-2-yl)-prop-2-yl]-2-[2-(4-fluorphenyl)-ethyl]-oxiran (60 %ig) in 600 ml n-Butanol gibt man 2,3 g gepulvertes Kaliumhydroxid und erhitzt 16 Stunden auf Rückflußtemperatur. Zur Aufarbeitung engt man das Reaktionsgemisch unter vermindertem Druck ein, nimmt den Rückstand in 700 ml Dichlormethan auf, wäscht zweimal mit jeweils 1500 ml Wasser, trocknet über

Natriumsulfat, engt unter vermindertem Druck ein und chromatographiert den Rückstand über Kieselgel (Laufmittel: Cyclohexan/Essigester 4:1).

Man erhält 124 g (86 % der Theorie) an 2-(1,3-Dioxolan-2-yl)-5-(4-fluorphenyl)-2-methyl-3-(1,2,4-triazol-1-ylmethyl)-pentan-3-ol als Öl.

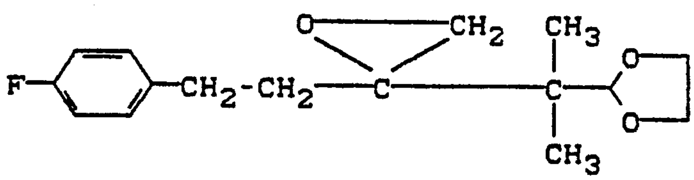

Zu 74,4 g (1,2 Mol) Dimethylsulfid in 350 ml absolutem Dimethylsulfoxid und 200 ml abolutem Tetrahydrofuran tropft man unter Rühren 170,4 g (1,2 Mol) Methyliodid. Man rührt das Reaktionsgemisch 16 Stunden bei Raumtemperatur, gibt dann tropfenweise unter Rühren 190 g (0,7 Mol) 2-(1,3-Dioxolan-2-yl)-5-(4-fluorphenyl)-2-methyl-pentan-3-on in 300 ml Toluol zu und dann bei 5° C portionsweise in einem Zeitraum von 90 Minuten in 3 Portionen 40 g (0,7 Mol) Natriummethylat. Man rührt weitere 3 Stunden bei Raumtemperatur, gibt dann bei 5° C innerhalb von 30 Minuten weitere 30 g (0,55 Mol) Natriummethylat in 2 Portionen zu, rührt 10 Stunden bei 20° C und weitere 8 Stunden bei 45° C. Danach engt man unter vermindertem Druck ein, nimmt den Rückstand in 1000 ml Dichlormethan auf, wäscht 3 mal mit jeweils 1000 ml Wasser, trocknet über Natriumsulfat und engt unter vermindertem Druck ein.

Man erhält 193 g (60 % der Theorie) an rohem 2-[2-(1,3-Dioxolan-2-yl)-prop-2-yl]-2-[2-(4-fluorphenyl)-ethyl]-oxiran in 60 %iger Reinheit. Das Produkt kann ohne zusätzliche Reinigungsschritte in die nächste Stufe eingesetzt werden.

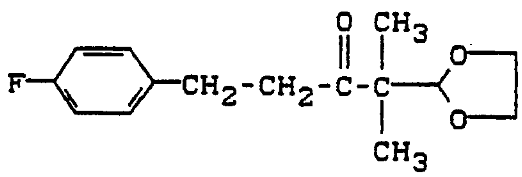

193 g (0,73 Mol) 2-(1,3-Dioxolan-2-yl)-5-(4-fluorphenyl)-2-methyl-pent-4-en-3-on in 1200 ml Tetrahydrofuran werden in Gegenwart von 20 g Raney-Nickel 7 Stunden bei 31° C und 55 bar Wasserstoffdruck hydriert. Zur Aufarbeitung wird der Katalysator abfiltriert und das Lösungsmittel abdestilliert.

Man erhält 190 g (99 % der Theorie) an 2-(1,3-Dioxolan-2-yl)-5-(4-fluorphenyl)-2-methyl-pentan-3-on als Öl, welches laut gaschromatographischer Analyse in 97 %iger Reinheit vorliegt.

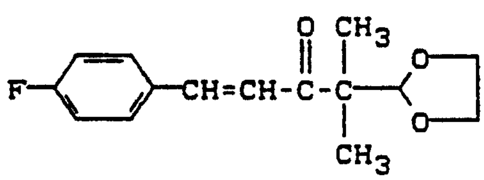

Zu 124 g (1 Mol) 4-Fluorbenzaldehyd und 158 g (1 Mol) 2-(1,3-Dioxolan-2-yl)-2-methyl-butan-3-on (vergl. z.B. DE-OS 32 42 252 oder DE-OS 32 42 236) in 350 ml Ethanol und 124 ml Wasser gibt man tropfenweise unter Rühren 84 ml 10%ige wässrige Natronlauge. Man rührt das Reaktionsgemisch nach beendeter Zugabe weitere 16 Stunden bei Raumtemperatur, saugt den ausgefallenen Feststoff ab und wäscht mit wenig Ethanol nach.

Man erhält 206 g (78 % der Theorie) an 2-(1,3-Dioxolan-2-yl)-5-(4-fluorphenyl)-2-methyl-pent-4-en-3-on vom Schmelzpunkt 76° C. Der Stoff kann aus Petrolether umkristallisiert werden.

Nach der im Beispiel 1 angegebenen Methode und gemäß den allgemeinen Angaben zur Herstellung erhält man die folgenden substituierten Triazolylalkanole der Formel (I):

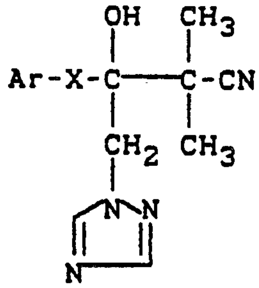

(I)

| Bsp. Nr. | Ar | X | physikalische Konstanten |
|---|---|---|---|
| 2 | 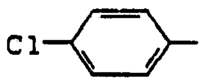 | $-CH_2-CH_2-$ | Fp: 141° C |
| 3 | 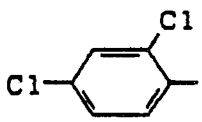 | $-CH_2-CH_2-$ | Fp: 102-104° C |
| 4 | 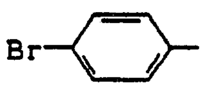 | $-S-CH_2-$ | $^1$H-NMR*): 3,24 (q); 4,58 (q) |
| 5 | 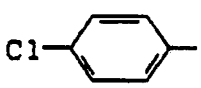 | $-S-CH_2-$ | $^1$H-NMR*): 3,23 (q); 4,56 (q) |
| 6 | 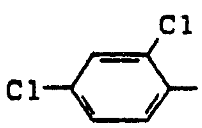 | $-O-CH_2-$ | Fp: 130-132° C |
| 7 | 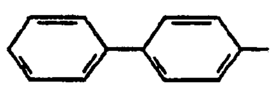 | $-O-CH_2$ | $^1$H-NMR*): 3,72 (q); 4,71 (q) |
| 8 | 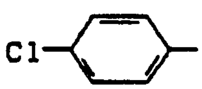 | $-O-CH_2-$ | Fp: 120-122° C |

| Bsp. Nr. | Ar | X | physikalische Konstanten |
|---|---|---|---|
| 9 | 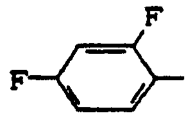 | $-CH_2-CH_2-$ | Fp: 133-135° C |
| 10 | 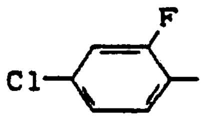 | $-CH_2-CH_2-$ | Fp: 123° C |
| 11 | 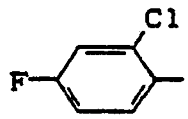 | $-O-CH_2-$ | Fp: 133-135° C |
| 12 | 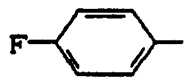 | $-S-CH_2-$ | $^1$H-NMR*): 3,2 (q); 4,55 (q) |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichssubstanzen eingesetzt:

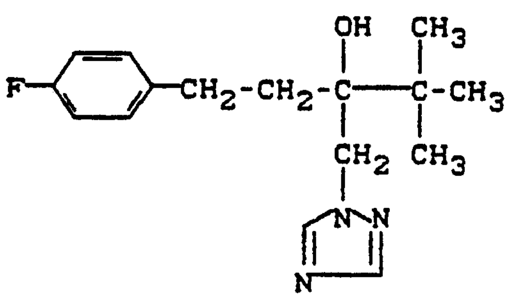

(A)

2,2-Dimethyl-5-(4-fluorphenyl)-3-(1,2,4-triazol-1-ylmethyl)-pentan-3-ol
(bekannt aus DE-OS 30 18 866)

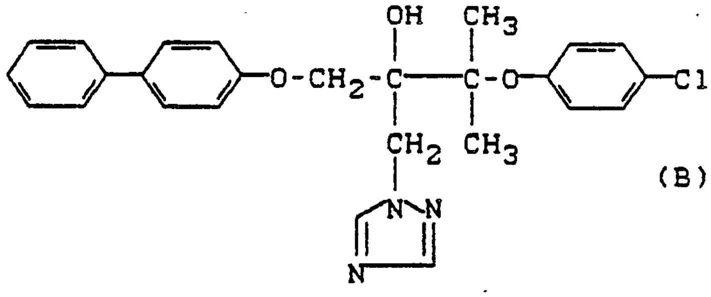

(B)

3-(4-Chlorphenoxy)-3-methyl-1-(4-phenylphenoxy)-2-(1,2,4-triazol-1-ylmethyl)-butan-2-ol
(bekannt aus DE-O 32 02 601)

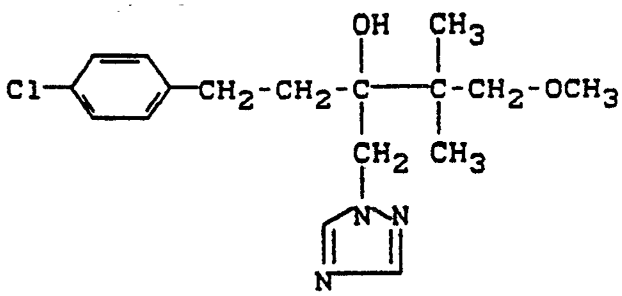

(C)

5-(4-Chlorphenyl)-2,2-dimethyl-1-methoxy-3-(1,2,4-triazol-1-ylmethyl)-pentan-3-ol
(bekannt aus DE-OS 32 02 601)

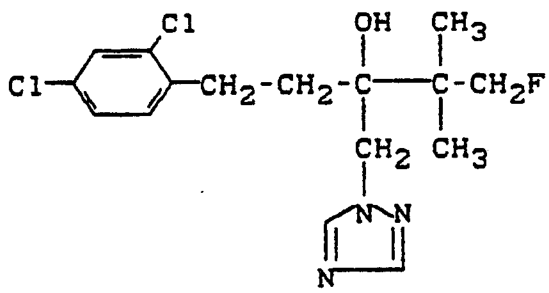

(D)

5-(2,4-Dichlorphenyl)-2,2-dimethyl-1-fluor-3-(1,2,4-triazol-1-ylmethyl)-pentan-3-ol
(bekannt aus DE-OS 32 02 601)

Beispiel A

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

In diesem Test zeigen die in den Beispielen 3, 7, 10 und 11 beschriebenen erfindungsgemäßen Stoffe eine wesentlich bessere Wirksamkeit als die Vergleichssubstanzen (A) und (B).

Beispiel B

Sphaerotheca-Test (Gurke) / protektiv

Lösungsmittel: 4.7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

In diesem Test zeigen die in den Beispielen 6, 7, 8 und 11 beschriebenen erfindungsgemäßen Stoffe eine wesentlich bessere Wirksamkeit als die Vergleichssubstanz (B).

Beispiel C

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

In diesem Test zeigt die in dem Beispiel 2 beschriebene erfindungsgemäße Verbindung eine wesentlich bessere Wirkung als die Vergleichssubstanzen (C) und (D).

Beispiel D

Wachstum bei Sojabohnen

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte

Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeuten 100 % Wachstum ein Wachstum entsprechend dem der Kontrollpflanzen und 0 % den Stillstand des Wachstums. Werte über 100 % kennzeichnen eine Wuchsförderung.

In diesem Test zeigen die in den Beispielen 3, 6, 8, 9, 10 und 11 beschriebenen erfindungsgemäßen Stoffe eine sehr starke wuchshemmende Wirkung.

Beispiel E

Wachstum bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 2 Wochen wird der Zuwachs der Pflanzen gemessen und die Wuchshemmung in Prozent des Wachstum der Kontrollpflanzen berechnet. Es bedeuten 100 % Wachstum: ein Wachstum entsprechend dem der Kontrollpflanzen und 0 % den Stillstand des Wachstums. Werte über 100 % kennzeichnen eine Wuchsförderung.

In diesem Test zeigen die in dem Beispiel 8, beschriebene erfindungsgemäße Verbindung eine sehr starke wuchshemmende Wirkung.

Beispiel F

Wachstum bei Roggen

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Roggenpflanzen werden im Gewächshaus bis zur Zweiblatt-Stadium angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeuten 100 % Wachstum ein Wachstum entsprechend dem der Kontrollpflanzen und 0 % den Stillstand des Wachstums. Werte über 100 % kennzeichnen eine Wuchsförderung.

In diesem Test zeigt die in den Beispielen 8 beschriebene erfindungsgemäße Verbindung eine sehr starke wuchshemmende Wirkung.

## Ansprüche

1. Substituierte Triazolylalkanole der Formel

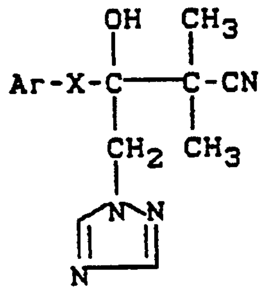

(I)

in welcher
Ar für gegebenenfalls substituiertes Aryl steht und
X für eine der Gruppen $-CH_2-$, $-O-CH_2-$, $-S-CH_2-$, $-CH_2-CH_2-$, $-CH=CH-$ oder $-C\equiv C-$ steht,
sowie deren Säureadditionssalze und Metallsalzkomplexe.

2. Substituierte Triazolylalkanole der Formel (I) gemäß Anspruch 1, in denen
Ar für Phenyl steht, das einfach oder mehrfach, gleichartig oder verschieden substituiert sein kann durch Halogen, Nitro, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Halogenalkylthio mit 1 bis 4 Kohlenstoffatomen und 1 bis 9 gleichen oder verschiedenen Halogenatomen, durch gegebenenfalls ein- oder mehrfach, gleichartig oder verschieden durch Halogen substituiertes Phenyl und/oder gegebenenfalls ein- oder mehrfach, gleichartig oder verschieden durch Halogen substituiertes Phenoxy, oder
Ar für gegebenenfalls einfach oder mehrfach, gleichartig oder verschieden durch Halogen und/oder Alkyl mit 1 bis 4 Kohlenstoffatomen substituiertes Naphthyl steht und
X für die Gruppierungen $-CH_2-$, $-OCH_2-$, $-SCH_2-$, $-CH_2-CH_2-$, $-CH=CH-$ oder $-C\equiv C-$ steht, wobei das Heteroatom mit dem Arylrest verbunden ist, wenn X für $-OCH_2-$ oder $-SCH_2-$ steht.

3. Substituierte Triazolylalkanole der Formel (I) gemäß Anspruch 1, in denen
Ar für Phenyl steht, das einfach bis dreifach, gleichartig oder verschieden substituiert sein kann durch Fluor, Chlor, Brom, Nitro, Methyl, Ethyl, n- oder i-Propyl, n-, i-, s-, oder t-Butyl, Methoxy, Ethoxy, n- oder i-Propoxy, Methylthio, Trifluormethyl, Trifluormethoxy, Trifluormethylthio, gegebenenfalls einfach bis dreifach, gleichartig oder verschieden durch Fluor, Chlor und/oder Brom substituiertes Phenyl und/oder gegebenenfalls einfach bis dreifach, gleichartig oder verschieden durch Fluor, Chlor und/oder Brom substituiertes Phenoxy, oder
Ar für gegebenenfalls einfach bis dreifach, gleichartig oder verschieden durch Fluor, Chlor, Brom und/oder Methyl substituiertes Naphthyl steht und
X für die Gruppierungen $-CH_2-$, $-OCH_2-$, $-SCH_2-$, $-CH_2-CH_2-$, $-CH=CH-$ oder $-C\equiv C-$ steht, wobei das Heteroatom mit dem Arylrest verbunden ist, wenn X für $-OCH_2-$ oder $-SCH_2-$ steht.

4. Verfahren zur Herstellung von substituierten Triazolylalkanolen der Formel

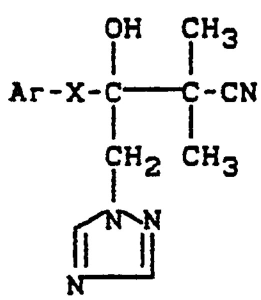

(I)

in welcher

Ar für gegebenenfalls substituiertes Aryl steht und
X für eine der Gruppen $-CH_2-$, $-O-CH_2-$, $-S-CH_2-$, $-CH_2-CH_2-$, $-CH=CH-$ oder $-C\equiv C-$ steht,
sowie von deren Säureadditionssalzen und Metallsalzkomplexen, dadurch gekennzeichnet, daß man Oxim-Derivate der Formel

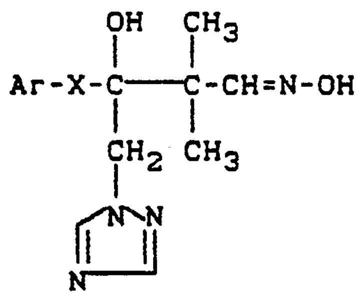

(II)

in welcher
Ar und X die oben angegebene Bedeutung haben,
mit einem wasserabspaltenden Mittel, gegebenenfalls in Gegenwart eines Verdünnungsmittels und gegebenenfalls eines Reaktionshilfsmittels, umsetzt und gegebenenfalls anschließend an die so erhaltenen substituierten Triazolylalkanole der Formel (I) eine Säure oder ein Metallsalz addiert.

5. Fungizide und pflanzenwuchsregulierende Mittel, gekennzeichnet durch einen Gehalt an mindestens einem substituierten Triazolylalkanol der Formel (I) gemäß Anspruch 1 bzw. an einem Säureadditionssalz oder Metallsalzkomplex eines substituierten Triazolylalkanols der Formel (I).

6. Verwendung von substituierten Triazolylalkanolen der Formel (I) gemäß Anspruch 1 bzw. von deren Säureadditionssalzen und Metallsalzkomplexen zur Bekämpfung von Pilzen sowie zur Regulierung des Pflanzenwachstums.

7. Verfahren zur Bekämpfung von Pilzen sowie zur Regulierung des Pflanzenwachstums, dadurch gekennzeichnet, daß man substituierte Triazolylalkanole der Formel (I) gemäß Anspruch 1 bzw. deren Säureadditions-Salze oder Metallsalzkomplexe auf die Pflanzen und/oder deren Lebensraum ausbringt.

8. Verfahren zur Herstellung von fungiziden und pflanzenwuchsregulierenden Mitteln, dadurch gekennzeichnet, daß man substituierte Triazolylalkanole der Formel (I) gemäß Anspruch 1 bzw. deren Säureadditionssalze oder Metallsalzkomplexe mit Streckmitteln und/oder oberflächenaktiven Stoffen vermischt.